# EUROPÄISCHE PATENTANMELDUNG

(11) **EP 0 805 146 A1**
(43) Veröffentlichungstag der Anmeldung: **05.11.1997**
(21) Anmeldenummer: 97107179.0
(22) Anmeldetag: 30.04.1997
(51) Int. Cl.: C07C 231/12, B01J 35/02

(54) **Katalysatorträger und Katalysatoren für Dehydrocyanisierungsreaktionen und deren Herstellungsverfahren**

(30) Priorität: 02.05.1996 DE 19617530; 06.05.1996 DE 19618129
(71) Anmelder: BASF AKTIENGESELLSCHAFT, 67056 Ludwigshafen (DE)
(72) Erfinder: Dupuis, Jacques, Dr., 67069 Ludwigshafen (DE); Trübenbach, Peter, Dr., 67065 Ludwigshafen (DE); Schwab, Ekkehard, Dr., 67434 Neustadt (DE); Kröner, Michael, Dr., 14558 Bergholz-Rehbrücke (DE)
(74) Vertreter: Isenbruck, Günter, Dr.

(57) **Zusammenfassung**

In dem SiO₂-freien, α-Al₂O₃ enthaltenden Katalysatorträger in Form eines Hohlkörpers ist mindestens ein Teil der Außenwände des Hohlkörpers durchbrochen. Umfaßt sind auch ein Verfahren zu seiner Herstellung, ein Katalysator aus obigem Träger, ein Verfahren zu dessen Herstellung, sowie dessen Verwendung für Dehydrocyanisierungsreaktionen.

## Beschreibung

Die vorliegende Erfindung betrifft einen SiO₂-freien, α-Al₂O₃ enthaltenden Katalysatorträger in Form eines Hohlkörpers, dadurch gekennzeichnet, daß mindestens ein Teil der Außenwände des Hohlkörpers durchbrochen ist. Ferner betrifft sie ein Verfahren zu seiner Herstellung, einen Katalysator, der obigen Träger umfaßt, dessen Herstellung sowie die Verwendung des Katalysators für Dehydrocyanisierungsreaktionen.

Aus der DE-A-195 33 486 und der DE-A-195 33 484 sind komplex geformte Katalysatorträger bzw. Katalysatoren und deren Herstellungsverfahren bekannt, die sich durch eine enge monomodale oder polymodale Porenverteilung auszeichnen.

N-Vinylformamid wird durch thermische Dehydrocyanisierung aus N-Formylalaninnitril mit einem Katalysator auf der Basis von Al₂O₃ hergestellt. Der bisher verwendete Katalysator wurde durch Tränkung eines Al₂O₃-Katalysatorträgers mit einer Aktivkomponente und nachfolgender Trocknung hergestellt. Als Katalysatorgeometrien wurden bisher Ringe, Wagenräder und Wabenkorper verwendet. Dabei wurde teilweise SiO₂ zugefügt, um die mechanische Festigkeit der Al₂O₃-Katalysatorträger zu erhöhen. So beschreibt die EP-A-0 206 193 (bzw. die DE-A 35 21 766) einen wabenförmigen Katalysator, enthaltend als Hauptbestandteile 30 bis 95 Gew.- % einer Eisenverbindung, berechnet als Fe₂O₃, und 0,1 bis 60 Gew.-% einer Aluminium-, Cer- und/oder Chromverbindung, berechnet als Al₂O₃, CeO₂, Cr₂O₃, und/oder 1 bis 50 Gew.-% einer Alkaliverbindung, sowie die Verwendung dieses Katalysators als Dehydrocyanisierungskatalysator.

Die EP-A-0 208 929 betrifft einen Katalysatorfestbettaufbau unter Verwendung des in den oben zitierten Anmeldungen beschriebenen Katalysators für exo- und endotherme chemische Reaktionen mit hoher Wärmetönung.

Die DE-A 34 43 463 betrifft ein Verfahren zur Herstellung von N-Vinylformamid durch Pyrolyse von Formylalaninnitril in Gegenwart von Feststoffen als Katalysatoren, wobei dort als Katalysatoren Alkali- und Erdalkalicarbonate, Magnesiumoxid, Calciumoxid, Bariumoxid und - als besonders vorteilhaft - Katalysatoren, die Alkali- und/oder Erdalkalicarbonate auf α-Aluminiumoxid als Träger enthalten, verwendet werden.

Während der katalytischen Synthese von N-Vinylformamid ausgehend von N-Formylalaninnitril reagieren die Aktivkomponenten, wie z.B. Alkalimetallverbindungen und/oder Erdalkalimetallverbindungen, mit SiO₂ bei Temperaturen von 300 °C bis 600 °C allmählich zu Alkali- und/oder Erdalkalimetallsilikaten, die bezüglich der zu katalysierenden Reaktion inaktiv sind und deren fortschreitende Bildung die mechanische Festigkeit des Katalysators verringert. Bei der Verwendung der herkömmlicherweise verwendeten, SiO₂ enthaltenden Katalysatoren geht demnach während der Reaktion laufend Aktivkomponente verloren und ferner verringert sich mit fortschreitender Reaktion die Stabilität des Katalysators.

Durch Extrusion können SiO₂-freie Rohre oder Wagenräderprofile hergestellt werden. Über Preßverfahren sind bislang nur SiO₂-haltige Formkörper herzustellen, da die SiO₂-freien gepreßten Aluminiumoxidformkörper aufgrund ihrer relativ geringen mechanischen Festigkeit nach dem Sintern zerfallen. Ohne Nachbearbeitung sind demnach nur einfache Geometrien von SiO₂-freien Al₂O₃-Formkörpern über die Extrusion in Rohr- oder Strangform zugänglich. Bei Verwendung einfacher Katalysatorgeometrien resultieren aufgrund ungünstiger Durchströmung der Reaktorschüttung bei katalytischen Reaktionen, wie z.B. Dehydrocyanisierungsreäktionen, hohe Druckverluste, was zu unbefriedigenden Durchsätzen führt.

Bei diesen stark exothermen Reaktionen ist es darüber hinaus wichtig, daß ein hoher Wärmeeintrag in das Katalysatorschüttgut gewährleistet ist. Bei Reaktionen unterhalb von Normaldruck wird der Wärmeeintrag vorwiegend durch Wärmestrahlung und nicht durch Wärmeleitung gewährleistet. Übliche Geometrien von Katalysatorträgern, wie z.B. Ringe, Wagenräder und Wagenkörper, stellen jedoch Hindernisse für die Wärmeübertragung durch Strahlung dar.

Der Erfindung lag somit die Aufgabe zugrunde, einen SiO₂-freien α-Al₂O₃ enthaltenden Katalysatorträger, bei dessen Anwendung ein geringerer Druckverlust, ein höherer Wärmeeintrag, kein Aktivkomponentenverlust und kein Verlust der mechanischen Stabilität während der katalytischen Reaktion, insbesondere bei Dehydrocyanierungsreaktionen, auftritt, bereitzustellen.

Diese Aufgabe wurde durch den erfindungsgemäßen Katalysatorträger, bzw. Katalysator gelöst.

Wie eingangs erwähnt betrifft die vorliegende Erfindung einen SiO₂-freien, α-Al₂O₃ enthaltenden Katalysatorträger in Form eines Hohlkörpers, dadurch gekennzeichnet, daß mindestens ein Teil der Außenwände des Hohlkörpers durchbrochen ist.

Ferner betrifft die Erfindung einen SiO₂-freien, α-Al₂O₃ enthaltenden Katalysator, umfassend einen Katalysatorträger, wie oben definiert, und, darauf aufgebracht, mindestens eine Alkalimetallverbindung oder mindestens eine Erdalkalimetallverbindung oder ein Gemisch davon.

Unter die Definition der Form des erfindungsgemäßen Katalysatorträgers fallen alle Hohlkörper, wie z.B. Ringe, Wagenräder und Wabenkörper, aber auch Kugeln, Würfel, Quader, Zylinder, Pyramiden- und Kegelstümpfe, Tetraeder mit abgeschnittener Spitze, wobei jedoch erfindungsgemäß mindestens ein Teil der Außenwände durchbrochen ist.

Vorzugsweise erstreckt sich der Katalysatorträger entlang einer Längsachse und verjüngt sich entlang derselben. Dabei ist zu beachten, daß vorzugsweise die Durchbrechungen der Außenwände des Hohlkörpers parallel zur Längsachse orientiert sind. Unter diesen sich verjüngenden Formen sind insbesondere Kegelstumpf, Tetraeder mit abgeschrittener Spitze, Pyramidenstumpf, sowie pyramidenstumpfähnliche Formen zu nennen. Der Begriff "pyramidenstumpfähnliche Formen" bezeichnet im Kontext der vorliegenden Anmeldung alle Formen, deren Grund- und Deckplatte, im Unterschied zum Pyramidenstumpf, nicht quadratisch sondern rechteckig sind. In seiner extremsten Ausgestaltung läßt sich der Katalysatorträger in pyramidenstumpfähnlicher Form durch zwei Fensterrahmen beschreiben, die durch mindestens drei Säulen miteinander verbunden sind, wobei einer der Fensterrahmen (Grundplatte) eine größere Kantenlänge als der andere (Deckplatte) besitzt. Weiter bevorzugt liegt der Katalysatorträger in Form eines Kegelstumpfes, eines Pyramidenstumpfes und in pyramidenstumpfähnlicher Form vor.

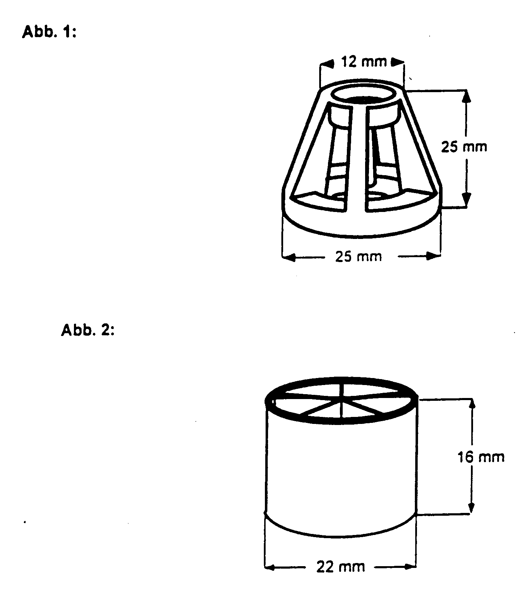

Abb. 1 zeigt eine schematische Darstellung des erfindungsgemäßen Katalysatorträgers in Form eines Kegelstumpfes. Verwendet man anstelle der beiden Ringe entsprechende quadratische bzw. rechteckige Grund- bzw. Deckrahmen entspricht diese Darstellung dem was im Rahmen der vorliegenden Anmeldung mit pyramidenstumpfförmig bzw. pyramidenstumpfähnlich gemeint ist.

Insbesondere bevorzugt sind Katalysatorträger in Form eines Kegelstumpfes mit folgenden Dimensionen:
a) Grundringdurchmesser:
   ungefähr 10 bis ungefähr 300 mm, vorzugsweise ungefähr 10 bis ungefähr 200 mm, weiter bevorzugt ungefähr 10 bis ungefähr 50 mm;
b) Höhe:
   ungefähr 10 bis ungefähr 300 mm, vorzugsweise ungefähr 10 bis ungefähr 200 mm, weiter bevorzugt ungefähr 10 bis ungefähr 50 mm;
c) Deckringdurchmesser:
   ungefähr 5 bis ungefähr 150 mm, vorzugsweise ungefähr 5 bis ungefähr 100 mm, weiter bevorzugt ungefähr 5 bis ungefähr 30 mm;
d) Anzahl der den Grund- und Deckring verbindenden Säulen:
   3 bis 12, wobei sich die Säulenzähl nach dem Durchmesser des Grund- und Deckrings richtet und bei Grunddurchmessern von 10 bis 50 mm 3 bis 4 beträgt, bei Grundringdurchmessern von ungefähr 20 bis ungefähr 100 mm 3 bis 6 und bei Durchmessern von ungefähr 100 bis 300 mm 3 bis 12 beträgt, wobei der Durchmesser der Säulen im allgemeinen bei ungefähr 0,5 bis ungefähr 50 mm liegt, wobei sich selbstverständlich der Durchmesser des Säulen nach der Größe des Grund- und Deckrings richtet.
und Katalysatorträger in Form eines Pyramidenstumpfes mit folgenden Dimensionen:
a) Kantenlänge der Grundplatte:
   ungefähr 7 bis ungefähr 200 mm, vorzugsweise ungefähr 7 bis unge fähr 150 mm, weiter bevorzugt ungefähr 7 bis ungefähr 40 mm;
b) Höhe:
   ungefähr 10 bis ungefähr 300 mm, vorzugsweise ungefähr 10 bis ungefähr 200 mm, weiter bevorzugt ungefähr 10 bis ungefähr 50 mm;
c) Kantenlänge der Deckplatte:
   ungefähr 5 bis ungefähr 150 mm, vorzugsweise ungefähr 5 bis ungefähr 100 mm, weiter bevorzugt ungefähr 5 bis ungefähr 25 mm;
d) Anzahl der die Grund- und Deckplatte verbindenden Säulen:
   3 bis 12, wobei sich die Säulenzahl nach der Kantenlänge der Grund- und Deckplatte richtet und bei Grundkantenlängen von ungefähr 10 bis ungefähr 50 mm 3 bis 4 beträgt, bei Grundkangenlängen von ungefähr 20 bis ungefähr 100 mm 3 bis 6 und bei Grundkantenlängen von ungefähr 100 bis ungefähr 300 mm 3 bis 12 beträgt, wobei der Durchmesser der Säulen im allgemeinen bei ungefähr 0,5 bis ungefähr 50 mm liegt, wobei sich selbstverständlich der Durchmesser der Säulen nach der Größe der Grund- und Deckplatte richtet.
   Der Durchmesser der oben beschriebenen Säulen beträgt vorzugsweise ungefähr 0,5 bis 30 mm, weiter bevorzugt ungefähr 0,5 bis 20 mm.

Obwohl das Verhältnis der Durchmesser zwischen Grund- und Deckring bzw. der Kantenlängen der Grund- und Deckplatte keinen besonderen Beschränkungen unterliegt, liegt das Verhältnis zwischen dem Durchmesser und der Kantenlänge des Grundrings bzw. der Grundplatte zu den Ausmaßen des entsprechenden Deckrings bzw. der entsprechenden Deckplatte vorzugsweise bei ungefähr 2:1.

Dabei ist zu beachten, daß die Säulen stab- oder treppenförmig mit einer oder mehreren Stufen ausgeführt sein können.

Ferner betrifft die vorliegende Erfindung einen SiO₂ freien, α-Al₂O₃ enthaltenden Katalysator, umlassend einen Katalysatorträger, wie oben erwähnt, und, darauf aufgebracht mindestens eine Alkalimetallverbindung oder mindestens eine Erdalkalimetallverbindung oder Gemisch davon.

Als Alkalimetallsalze bzw. Erdalkalimetallsalze zum Aufbringen der Aktivkomponente auf dem erfindungsgemäßen Katalysatorträger können prinzipiell alle wasserlöslichen Alkali- und/oder Erdalkalimetallsalze verwendet werden. Vorzugsweise werden jedoch die Carbonate und Acetate, wie z.B. Natriumcarbonat, Kaliumcarbonat, Magnesiumcarbonat, Strontiumcarbonat, Bariumcarbonat, Marmor, Dolomit, Kreide, Magnesit und Calciumacetat, sowie Gemische davon verwendet. Besonders bevorzugt wird Kaliumcarbonat verwendet.

Diese Alkali- und/oder Erdalkalimetallsalze werden bei der Trocknung des Katalysators in die entsprechenden Oxide und/oder Carbonate überführt.

Die spezifischen Oberflächen nach BET der erfindungsgemäßen Katalysatorträger und Katalysatoren liegen in der Regel bei ungefähr 0,01 bis ungefähr 100 m²/g, vorzugsweise ungefähr 0,1 bis ungefähr 50 m²/g, weiter bevorzugt bei ungefähr 0,5 bis ungefähr 10 m²/g und insbesondere bei ungefähr 1 bis ungefähr 8 m²/g.

Selbstverständlich können die Katalysatorträger und Katalysatoren auch mit anorganischen Pulvern oder Pulvermischungen durch Hicoaten beschichtet werden, wobei spezifische Oberflächen von über 100 m²/g erreicht werden können.

Die Porenverteilung innerhalb des erfindungsgemäßen Katalysatorträgers bzw. des erfindungsgemäßen Katalysators ist monomodal oder polymodal, d.h. bimodal, trimodal, tetramodal oder höhermodal, vorzugsweise monomodal oder bimodal, weiter bevorzugt monomodal, wobei der mittlere Porendurchmesser bei ungefähr 0,01 bis ungefähr 10,0 µm, vorzugsweise ungefähr 0,05 bis 1,0 µm, weiter bevorzugt bei ungefähr 0,1 bis ungefähr 0,8 µm, jeweils gemessen mit der Hg-Druckporosimetrie-Methode liegt.

Die hier in Rede stehenden Katalysatorträger können durch Pressen und insbesondere durch Spritzguß ohne Nachbearbeitung in einem Formgebungsschritt hergestellt werden. Mit dem erfindungsgemäßen Herstellungsverfahren ist es möglich, diese Katalysatorträger mit ausreichender mechanischer Stabilität SiO₂-frei herzustellen. Die aus reinem, d.h. mindestens 95%-igem, Al₂O₃ bestehenden Katalysatorträger erleiden nach dem Imprägnieren mit der Aktivkomponente keinen Aktivkomponentenverlust durch Silikatbildung, keinen Abbau der mechanischen Stabilität und erlauben demnach höhere Standzeiten bei höheren Durchsätzen.

Demgemäß betrifft die vorliegende Erfindung auch ein Verfahren zur Herstellung eines Katalysatorträgers, das die folgenden Stufen umfaßt:
I) Verformen eines Gemischs, enthaltend
   (A) ein Pulver mit einem Gehalt an α-Al₂O₃ von mindestens 95 Gew.-% und
   (B) ein polymeres Bindemittel
II) Entfernen des polymeren Bindemittels (B), und
III) Vorsintern des aus Stufe (II) erhaltenen Formkörpers.

Vorzugsweise enthält das in Stufe (I) verformte Gemisch zusätzlich als Komponente
C) ein Dispergierhilfsmittel, das in der Stufe (II) oder (III) entfernt wird.

Ferner betrifft die Erfindung ein Verfahren zur Herstellung eines Katalysators, das zusätzlich zu den Stufen I) bis III), wie oben definiert, die Stufe
IV) Imprägnieren des aus Stufe (III) erhaltenen Formkörpers mit mindestens einer Lösung mindestens eines Alkalimetallsalzes oder mindestens eines Erdalkalimetallsalzes oder eines Gemisches davon und anschließendes Trocknen des erhaltenen Katalysators
umfaßt, sowie einen SiO₂-freien, α-Al₂O₃ enthaltenden Katalysator, umfassend einen Katalysatorträger und mindestens eine Alkalimetallverbindung oder mindestens eine Erdalkalimetallverbindung oder Gemische davon, herstellbar durch ein Verfahren, das die folgenden Stufen umfaßt:
I) Verformen eines Gemischs, enthaltend
   (A) ein Pulver mit einem Gehalt an α-Al₂O₃ von mindestens 95 Gew.-% und
   (B) ein polymeres Bindemittel
II) Entfernen des polymeren Bindemittels (B),
III) Vorsintern des aus Stufe (II) erhaltenen Formkörpers, und
IV) Imprägnieren des aus Stufe (III) erhaltenen Formkörpers mit mindestens einer Lösung mindestens eines Alkalimetallsalzes oder mindestens eines Erdalkalimetallsalzes oder eines Gemisches davon und anschließendes Trocknen des erhaltenen Katalysators.

Vorzugsweise wird das erfindungsgemäße Verfahren zur Herstellung eines Katalysatorträgers (unter Weglassung der Stufe IV) bzw. des Katalysators wie folgt durchgeführt:
I) Verformen eines Gemisches, enthaltend
   (A) 50 bis 70 Vol.-% eines Pulvers, enthaltend mindestens 95 Gew.-% Al₂O₃
   (B) 30 bis 85 Vol.-% eines polymeren Bindemittels, das ausgewählt wird aus der Gruppe bestehend aus Polyethylenpolymeren, Polypropylenpolymeren, Copolymeren aus Ethylen, Propylen, Buten-1 oder Isobuten, Polystyrolcopolymeren, Polymethylmethacrylatcopolymeren, Polyethylenoxidcopolymeren, Ethylvinylacetatcopolymeren, sowie Gemischen daraus, und
   (C) 0 bis 15 Vol.- % eines Dispergierhilfsmittels,
II) Entfernen der Komponente (B) und - falls vorhanden - der Komponente (C) durch Pyrolyse bei Temperaturen von 400 bis 600 °C,
III) Vorsintern bei Temperaturen von 600 °C bis 1700 °C, und
IV) Aufbringen einer Aktivkomponente durch Tränken, Imprägnieren oder Sprühimprägnieren und anschließende Trocknung des so erhaltenen Katalysators.

Weiter bevorzugt wird im obigen Verfahren als Komponente (B) ein Gemisch, enthaltend
B₁) 50 bis 100 Gew.-% eines Polyoxymethylerhomo- oder -copolymers und
B₂) 0 bis 50 Gew.-% eines in B₁) homogen löslichen oder mit einer mittleren Teilchengröße von weniger als 1 µm in B₁) dispergierbaren Polymerisats durchgeführt, verwendet,
wobei bei Verwendung dieses Gemischs als Komponente (B) das Bindemittel nicht nur durch die oben definierte Pyrolyse, sondern auch durch säurekatalytische Entbinderung bei Temperaturen zwischen 100 °C und 150 °C in einer Inertgasatmosphäre durchgeführt werden kann.

Bei der säurekatalytischen Entbinderung der Komponente (B) wird die Komponente (C) im Rahmen des erfindungsgemäßen Verfahrens entweder in einer zusätzlichen Stufe (IIa) durch Pyrolyse entfernt oder während des Aufheizvorgangs der Vorsinterung gemäß Stufe (III) pyrolytisch bei ungefähr 300 bis ungefähr 600°C entfernt.
Das erfindungsgemäße Herstellungsverfahren soll im folgenden sowohl bezüglich der Reaktionsführung als auch der verwendeten Edukte näher erläutert werden.

Die Verformung kann wie folgt durchgeführt werden:
In einer Mischvorrichtung mit Heizvorrichtung, beispielsweise einem Kneter, einem Extruder oder einem Scherwalzenextruder kann dem Polymer der Komponente (B) in geschmolzenem Zustand bei Temperaturen von ungefähr 80 bis ungefähr 250 °C, vorzugsweise ungefähr 100 bis ungefähr 220 °C, weiter bevorzugt bei ungefähr 120 bis ungefähr 200 °C, die Komponente (A) und ggf. anschließend das Dispergierhilfsmittel (C) oder zuerst Komponente (C) und dann Komponente (A) oder die Komponenten (A) und (C) gemeinsam zugegeben werden. Die intensiv vermischten Massen können z.B. durch Extrusion, Pressen oder Spritzgießen, insbesondere durch Spritzgießen bei Temperaturen von ungefähr 120 bis ungefähr 250 °C, vorzugsweise ungefähr 140 bis ungefähr 220 °C, weiter bevorzugt bei ungefähr 150 bis ungefähr 200 °C und Drücken von ungefähr 500 bis ungefähr 2.000 bar, vorzugsweise ungefähr 600 bis ungefähr 1.800 bar, weiter bevorzugt bei ungefähr 700 bis ungefähr 1.600 bar verformt werden. Dabei können in einem Formgebungsschritt bei Spritzgießformtemperaturen von ungefähr 40 bis ungefähr 160 °C, vorzugsweise ungefähr 60 bis ungefähr 150 °C, weiter bevorzugt ungefähr 80 bis ungefähr 140 °C, beliebig geformte Katalysatorträger der hier in Rede stehenden Art ohne Nachbearbeitung geformt werden.

Die nach dem Formgebungsvorgang erhaltenen Grünkörper können durch Pyrolyse bei ungefähr 300 bis ungefähr 600 °C, vorzugsweise ungefähr 350 bis ungefähr 600 °C, weiter bevorzugt ungefähr 400 bis ungefähr 600 °C behandelt werden, wobei das Bindemittel (B) und die ggf. vorhandenen Dispergierhilfsmittel (C) entfernt werden.

Bei der Verwendung der bevorzugt eingesetzten Bindemittel B₁ und B₂, wie oben definiert, können diese in einer gasförmigen, säurehaltigen Atmosphäre bei Temperaturen von ungefähr 100 bis ungefähr 160 °C, vorzugsweise bei ungefähr 100 bis ungefähr 150 °C, weiter bevorzugt unter der Erweichungstemperatur des verwendeten Polyacetals (Komponente B₁ und B₂) katalytisch entbindert werden.

Der Begriff "*gasförmige, säurehaltige Atmosphäre*" bezeichnet hier sowohl reine Säuren, die bei den Behandlungstemperaturen gasförmig vorliegen, als auch Gemische von Säuren mit einem Trägergas. Als Trägergase kommen beispielsweise Luft oder Stickstoff oder Edelgase in Betracht.

Als Säuren kommen anorganische Säuren in Betracht, die bei Raumtemperatur gasförmig sind, wie z.B. Halogenwasserstoffe, Schwefelwasserstoffe oder solche Säuren, die bei den Behandlungstemperaturen im merklichen Umfang verdampfbar sind, wie z.B. Salpetersäure.

Als organische Säuren kommen grundsätzlich solche Säuren oder Säuregemische in Betracht, die einen Siedepunkt oder Sublimationspunkt bei Normaldruck von unter 130 °C haben, wie z.B. Oxalsäure, Ameisensäure, Essigsäure oder Trifluoressigsäure.

Anstelle der gasförmigen, säurehaltigen Atmosphäre können die entformten Grünkörper auch in einer gasförmiges Bortrifluorid enthaltenden Atmosphäre zwecks Entfernung des Bindemittels behandelt werden. Unter gasförmiges Bortrifluorid enthaltende Atmosphären kann sowohl reines Bortrifluorid als auch Gemische von Bortrifluorid mit einem Trägergas verstanden werden. Als Trägergas kommen beispielsweise Luft oder Stickstoff oder Edelgase in Betracht.

Anstelle von Bortrifluorid können selbstverständlich auch Addukte von Bortrifluorid verwendet werden, die bei den Behandlungstemperaturen reversibel und ohne Zersetzung der Komponenten wieder in die Ausgangskomponenten gespalten werden können. Insbesondere geeignet sind die Additionsverbindungen des Botrifluorids mit Ether, z.B. Dimethylether, Diethylether, Dibutylether und tert.-Butylmethylether.

Besonders bevorzugt sind Salpetersäure, wasserfreie Oxalsäure oder Oxalsäuredihydrat. Weiter eignet sich Glyoxalsäure. Außerdem kommen Benzolsulfonsäure, Naphthalinsulfonsäuren und Maleirsäure oder Gemische dieser Säuren in Betracht. Diese können sowohl für sich allein oder auch zusammen mit einem Trägergas wie Luft, Stickstoff oder einem Edelgas bei der Entbinderung eingesetzt werden.

Zur Erleichterung der Dosierung kann es zweckmäßig sein, die o.g. Säuren als Lösung in polaren Lösungsmitteln, vorzugsweise mit Siedepunkten unter 200 °C, einzusetzen. Als solche kommen vor allem Wasser, Isopropanol, Aceton, Dioxan, Ethanol, Essigsäure und Ameisensäure in Betracht. Die säurekatalysierte Entbinderung kann bei Normaldruck oder bei vermindertem Druck (ungefähr 0,001 bis ungefähr 1 bar) durchgeführt werden.

Die entweder durch Pyrolyse oder in einer gasförmigen, säurehaltigen Atmosphäre entbinderten und somit vom Bindemittel (B) befreiten Formkörper können durch anschließendes Vorsintern, in der Regel bei Temperaturen von ungefähr 600 bis ungefähr 1.600 °C, bevorzugt ungefähr 800 bis ungefähr 1400 °C, weiter bevorzugt ungefähr 900 bis ungefähr 1.300 °C unter oxidierenden Bedingungen, d.h. in Anwesenheit von Luft oder einem Inertgas, wie z.B. N₂ und AR, oder reduzierenden Bedingungen, d.h. in Anwesenheit von N₂, H₂, AR/H₂, zu den Katalysatorträgern in ihrer endgültigen Festigkeit und Porenverteilung gesintert werden.

Durch den Sinterprozeß erhöht sich in der Regel die Stabilität und die Härte der porösen Formkörper beträchtlich. Die Seitendruckfestigkeit der bei ungefähr 1.100 °C vorgesinterten Proben beträgt in der Regel ungefähr 100 bis ungefähr 1.000 N, vorzugsweise ungefähr 100 bis ungefähr 600 N, weiter bevorzugt ungefähr 200 bis ungefähr 500 N und insbesondere ungefähr 250 bis ungefähr 400 N. Die Wasseraufnahme liegt in der Regel im Bereich von ungefähr 0,05 bis ungefähr 5 ml/g, vorzugsweise bei ungefähr 0,1 bis ungefähr 1 ml/g, weiter bevorzugt bei ungefähr 0,1 bis ungefähr 0,5 ml/g.

Durch die ausgezeichnete Aktivkomponentenaufnahme können die Katalysatoren nach Gebrauch durch Nachtränken der Aktivkomponenten gut recycliert werden. Neben streng monomodalen Porengrößenverteilungen können so auch polymodale (bimodale, trimodale, tetramodale oder höhermodale) Porengrößenverteilungen hergestellt werden. Wie oben erwähnt, werden die vom Bindemittel (B) befreiten Formkörper lediglich vorgesintert, d.h. der Sinterungsprozeß wird vor der vollständigen Sinterung, die zu überaus dichten, nicht-porösen Formkörpern führt, bei dem gewünschten Porösitätsgrad, d.h. der gewünschten spezifischen Oberfläche gestoppt.

Die mittlere Porengröße wird in der Regel von der Korngröße der Komponente (A), d.h. nur durch die Zwischenräume der eingesetzten Pulverteilchen bestimmt. Die mittlere Porengröße und die Porengrößenverteilung sind deshalb von der mittleren Korngröße und der Teilchengrößenverteilung des eingesetzten Pulvers abhängig. Mit kommerziell erhältlichen Keramikpulvern können auf diese Weise mechanisch stabile, rißfreie, monomodal oder polymodal poröse Materialien, wie die erfindungsgemäßen Katalysatorträger oder Katalysatoren, hergestellt werden. Die enge Porengrößenverteilung kann somit je nach Bedarf im Meso- und Makroporenbereich eingestellt werden und führt in der Regel zu einer hoch monodispersen Porenverteilung.

Die mittlere Korngröße der erfindungsgemäß eingesetzten Pulver der Komponente (A) beträgt im allgemeinen ungefähr 0,01 bis ungefähr 500 µm, vorzugsweise ungefähr 0,3 bis ungefähr 100 µm, weiter bevorzugt ungefähr 0,5 bis ungefähr 10 µm.

Der erfindungsgemäße Katalysatorträger bzw. Katalysator hat den Vorteil, daß er im wesentlichen mikroporenfrei ist. In den Mikroporen werden häufig Nebenprodukte gebildet, da dort die Verweilzeit der Edukte größer als in den Meso- bzw. Makroporen ist. Als Komponente (A) eignet sich α-Aluminiumoxid mit einem Gehalt an reinem α-Aluminiumoxid von ungefähr 95 bis 100 Gew.-%. Das eingesetzte α-Al₂O₃ kann bis zu 5% Dotierhilfsmittel erhalten. Als Dotierhilfsmittel können genannt werden: Oxide der Elemente Kalium, Rubidium, Cäsium, Magnesium, Calcium, Strontium, Barium, Titan, Vanadin, Chrom, Mangan, Eisen, Kobalt, Nickel, Kupfer, Zink, Zirkonium, Niob, Molybdän, Rhutenium, Rhodium, Palladium, Silber, Cadmium, Hafnium, Tantal, Wolfram.

Als Komponente (B) werden vorzugsweise die folgenden verwendet:

Polyethylenpolymere, Polypropylenpolymere, Copolymere aus Ethylen, Propylen, Buten-1 oder Isobuten, Polystyrolcopolymere, Ethylvinylacetatcopolymere, Gemische aus
B₁) 50 bis 100 Gew.-%, vorzugsweise 70 bis 90 Gew.-%, weiter bevorzugt 80 bis 88 Gew.-% eines Polyoxymethylerhomo- oder -copolymerisats, wie sie aus der EP-A-0 444 475 bekannt sind und
B₂) 0 bis 50 Gew.-%, vorzugsweise 10 bis 30 Gew.-%, weiter bevorzugt 12 bis 25 Gew.-% eines in B₁) homogen gelösten oder mit einer mittleren Teilchengröße von weniger als 1 µm in B₁) dispergierten Polymerisats, vorzugsweise Poly-1,3-dioxolan, Poly-1,3-dioxan, Poly-1,3-dioxepan, wobei besonders bevorzugt Poly-1,3-dioxepan eingesetzt wird.

Das organische Bindemittel kann auch ein Gemisch eines oder mehrerer thermoplastischer Harze, wie z.B. Polyacetal, Polyethylen, Polypropylen, Polystyrol, Polymethylmethacrylat und ein oder mehrerer Weichmacher, wie z.B. Polyethylenglykol, Polypropylenglykol, Polybutandiolformal, Phthalsäureester, Ethylenvinylacetat-Copolymere und Montanesterwachse sein.

Als Polyacetalbindemittel eignen sich beispielsweise Polyoxymethylen, das vorzugsweise eine Molmasse von ungefähr 10.000 bis ungefähr 500.000 aufweist. Neben Homopolymeren des Formaldehyds oder Trioxans kommen auch Copolymere aus Trioxan mit beispielsweise cyclischen Ethern, wie z.B. Ethylenoxid und 1,3-Dioxolan, oder Formalen, wie 1,3-Dioxepan, 1,3-Dioxan oder deren Gemische oder Homopolymere, ausgewählt unter Poly-1,3-dioxolan, Poly-1,3-dioxan oder Poly-1,3-dioxepan in Betracht, wobei die Mengen der Copolymeren im allgemeinen bei ungefähr 10 bis ungefähr 30 Gew.-% der Polymeren liegen.

Außerdem können die organischen Bindemittel Hilfsmittel, wie thermoplastische Bindemittel, wie z.B. Polyethylen, Polymethylmethacrylat oder Polyethylenoxid, und Dispergiermittel, wie z.B. Schmiermittel, wie z.B. Polyethylenglykol, Stearinsäure, Fettalkohole, Polyvinylpyrrolidon oder Polyvinylalkohol enthalten. Die Menge an derartigen Hilfsmitteln liegt in der Regel zwischen ungefähr 0,1 und ungefähr 12 Gew.-%, bezogen auf die Gesamtmasse.

Als Komponente (C) eignen sich Dispergierhilfsmittel, wie sie aus EP-A-444 475 bekannt sind. Beispielhaft zu nennen sind organische Carbonsäuren, Amine, Amide oder Maleinimide, Stearinsäure, Polyvinylpyrrolidon, Polyvinylalkohol, Polyethylenglykol, Polypropylenglykol, Polyethylenoxid und Montanwachse, vorzugsweise organische Carbonsäuren, Amine, Amide oder Maleinimide, Polyethylenglykol und Polyethylenoxid, insbesondere bevorzugt organische Carbonsäuren, Amine, Maleinimide, Polyethylenglykol und Polyethylenoxid.
Die zur Herstellung des erfindungsgemäßen Katalysatorträgers oder Katalysators eingesetzten Gemische enthalten in der Regel vorzugsweise ungefähr 15 bis ungefähr 70 Gew.-%, weiter bevorzugt ungefähr 30 bis ungefähr 70 Gew.-%, insbesondere ungefähr 50 bis ungefähr 65 Gew.- % der Komponente (A),
ungefähr 30 bis ungefähr 85 Gew.-%, vorzugsweise ungefähr 30 bis ungefähr 70 Gew.- % und insbesondere ungefähr 35 bis ungefähr 50 Gew.-% der Komponente (B), und
0 bis ungefähr 15 Gew.-%, vorzugsweise ungefähr 1 bis ungefähr 12 Gew.-%, insbesondere ungefähr 2 bis ungefähr 8 Gew.-% der Komponente (C).

Bei dem erfindungsgemäßen Herstellungsverfahren werden in der Regel mit Hilfe eines Dispergierhilfsmittels (C) die Komponenten (A) desagglomeriert und damit die einheitlich großen Ausgangspulverpartikel mit einem vergleichsweise hohen Füllgrad in ein organisches Bindemittel (B) eingearbeitet. Das organische Bindemittel füllt die in der Regel nahezu einheitlich großen und regelmäßig angeordneten Zwischerräume der Pulverpartikel. Die bei dem Ausgangspulver gemäß Komponente (A) durch Agglomeratbildung vorhandenen Makroporen im Bereich von ungefähr 100 µm werden durch die Desagglomerierung in der Regel abgebaut. Nach der Entfernung des organischen Bindemittels und des organischen Dispergiermittels, soweit vorhanden, bleiben bei der Verwendung eines Pulvers mit einer engen monomodalen Korngrößenverteilung streng einheitlich große Poren zwischen den Pulverteilchen zurück. In der Regel beträgt der mittlere Porendurchmesser 25% des mittleren Korndurchmessers des eingesetzten Pulvers. Bei Einsatz von Pulvern mit polymodaler Korngrößenverteilung oder bei Einsatz von porösen Pulvern können auch polymodale (bimodale, trimodale, tetramodale oder höhermodale) Porenverteilungen hergestellt werden, wobei die Porengröße durch die Zwischenräume zwischen den Pulverteilchen und durch die innere Porösität der Pulverteilchen bedingt ist.

Bezüglich weiterer Details zur Herstellung von Katalysatoren mit spezieller Porenverteilung wird auf die DE-A-195 33 486 und die DE-A-195 33 484 verwiesen, deren Inhalt bezüglich der Katalysatorherstellung hiermit in die vorliegende Anmeldung eingeführt wird.

Zur Herstellung des erfindungsgemäßen Katalysators wird in Stufe (IV) des obigen Verfahrens der Katalysatorträger nach an sich bekannter Weise mit wasserlöslichen Alkalimetall- und/oder Erdalkalimetallsalzen getränkt und durch thermische Nachbehandlung, d.h. Trocknen bei ungefähr 100°C bis ungefähr 400°C, vorzugsweise ungefähr 100°C bis ungefähr 200°C, in die entsprechenden Carbonate und/oder Oxide überführt.

Prinzipiell können zur Imprägnierung alle wasserlöslichen Alkalimetall- und/oder Erdalkalimetallsalze verwendet werden.

Vorzugsweise werden jedoch Carbonate oder Acetate, wie Natriumcarbonat, Kaliumcarbonat, Magnesiumcarbonat, Strontiumcarbonat, Bariumcarbonat, Marmor, Dolomit, Kreide, Magnesit und Calciumacetat oder deren Gemische verwendet.

Ferner betrifft die vorliegende Erfindung die Verwendung des erfindungsgemäßen Katalysators als Dehydrocyanisierungskatalysator, vorzugsweise als Katalysator für die Umsetzung von N-Formylalaninnitril zu N-Vinylformamid.

Dabei wird das Ausgangsprodukt, beispielsweise N-Formylalaninnitril durch Pyrolyse in Gegenwart des erfindungsgemäßen Katalysators unter vermindertem Druck, Temperaturen von im allgemeinen 250 bis 650 °C und anschließender Abkühlung des Pyrolyseproduktes und nachfolgender Isolierung des gewünschten Produktes, z. B. N-Vinylformamid durchgeführt.

Die genauen Verfahrensparameter für die thermische Dehydrocyanisierung von N-Formylalaninnitril zu N-Formylformamid sind in der DE-A 34 43 463 beschrieben, deren Inhalt hiermit in die vorliegende Anmeldung eingeführt wird.

### BEISPIELE

### Beispiel 1

1.000 g des α-Al₂O₃-Pulvers CT 3000 SG (ALCOA) wurden mit einem Bindemittel auf Polyacetalbasis, bestehend aus 162 g eines Polyoxymethylencopolymers (POM/PBDF) aus Trioxan und 2,5 Gew.- % Butandiolformal mit einem durchschnittlichen Molekulargewicht von 150.000, sowie mit 41 g Polybutandiolformal mit einem Molekulargewicht von 50.000 und 50 g Polyethylenglykol mit einem Molekulargewicht von 800 als Hilfsmittel, bei 180 °C verknetet, zu den in Abb. 1 gezeigten Katalysatorträgern mit einer Massetemperatur von 180 °C, einem Spritzdruck von 1.000 bar und einer Formtemperatur von 140 °C auf einer ALLROUNDER 370 C (ARBURG) spritzgegossen. Diese Katalysatorträger (25 mm x 25 mm x 12 mm, 4 Säulen, siehe Abb. 1) wurden bei 600 °C 2 h unter N₂ pyrolysiert und danach 2 h bei 1.100 °C im Muffelofen an Luft vorgesintert.

An den calcinierten Katalysatorträgern wurden folgende Eigenschaften gemessen:

| | |
|---|---|
| - Seitendruckfestigkeit | 298 N (±77 N) |
| - Wasseraufnahme | 0,176 ml/g |
| - BET | 4,49 m²/g |
| - mittlerer Porendurchmesser (nach der Hg-Druckporosimetrie-Methode) | 0,165 µm |
| - Gesamtporenvolumen (nach der Hg-Druckporosimetrie-Methode) | 0,185 ml/g |

16 kg der Katalysatorträger (25 mm x 25 mm x 12 mm, 4 Säulen, siehe Abb. 1) wurden mit 10 l einer 40%-igen wäßrigen Kaliumcarbonat-Lösung in einem Pyrolysereaktor (Durchmesser = 100 mm, Länge = 3 m) 12 h lang getränkt und bei 100 °C 12 h lang getrocknet. Danach wurden die so erhaltenen Katalysatoren bezüglich der Synthese von N-Vinylformamid aus N-Formylalaninnitril bei 10 mbar und 400 °C getestet.

| | |
|---|---|
| - Umsatz | 93% |
| - Selektivität | 98,8% |
| - Druckverlust | 2 mbar |
| - Kaliumverlust | 25% (nicht als Silikat) |
| - Wärmedurchgangszahl | 28 W/m²K |
| - radiale Wärmeleitfähigkeit | 0,3 W/mK |
| - Standzeit | >2.000 h |

### Beispiel 2

1.000 g des α-Al₂O₃-Pulvers wurden mit einem Bindemittel auf Polyacetalbasis, bestehend aus 162 g eines Polyoxymethylencopolymers (POM/PBDF) aus Trioxan und 2,5 Gew.- % Butandiolformal mit einem durchschrittlichen Molekulargewicht von 150.000, sowie mit 41 g Polybutandiolformal mit einem Molekulargewicht von 50.000 und 50 g Polyethylenglykol mit einem Molekulargewicht von 800 als Hilfsmittel, bei 180 °C verknetet, zu den in Abb. 1 gezeigten Katalysatorträgem mit einer Massetemperatur von 180 °C, einem Spritzdruck von 1.000 bar und einer Formtemperatur von 140 °C auf einer ALLROUNDER 370 C (ARBURG) spritzgegossen.

Diese Katalysatorträger (25 mm x 25 mm x 12 mm, 4 Säulen, siehe Abb. 1) wurden in einem HERAEUS-Entbinderungsofen (60 l) mit 70 ml/h einer 5 Gew.-%-igen Lösung von Oxalsäure in Essigsäure (99%-ig) oder 30 ml/h 100%-iger Salpetersäure bei 140 °C unter einem N₂-Strom von 300 l/h 10 Stunden lang entbindert, wobei das Polyoxymethylen zu Formaldehyd depolymerisiert. Danach wurden die Katalysatorträger im Muffelofen zunächst 2 h bei 600 °C an Luft und danach 2 h bei 1.300 °C an Luft calciniert.

An den calcinierten Katalysatorträgern wurden folgende Eigenschaften gemessen:

| | |
|---|---|
| - Seiterdruckfestigkeit | 650 N (±170 N) |
| - Wasseraufnahme | 0,109 ml/g |
| - BET | 2,85 m²/g |
| - mittlerer Porendurchmesser (nach der Hg-Druckporosimetrie-Methode) | 0,150 µm |
| - Gesamtporenvolumen (nach der Hg-Druckporosimetrie-Methode) | 0,127 ml/g |

Nach Tränkung und Trocknung der Katalysatorträger (25 mm x 25 mm x 12 mm, 4 Säulen, siehe Abb. 1) wie in Beispiel 1 wurden die so erhaltenen Katalysatoren wie in Beispiel 1 bezüglich ihrer Performance bei der Synthese von N-Vinylformamid aus N-Formylalaninnitril getestet.

| | |
|---|---|
| - Umsatz | 91% |
| - Selektivität | 98,7% |
| - Druckverlust | 2 mbar |
| - Kaliumverlust | 25% (nicht als Silikat) |
| - Wärmedurchgangszahl | 28 W/m²K |
| - radiale Wärmeleitfähigkeit | 0,3 W/mK |
| - Standzeit | >2.000 h |

### Vergleichsbeispiel

Die in Abb. 2 dargestellten Wagenräder-Katalysatorträger wurden in bekannter Weise nach dem Extrudierverfahren gefertigt. Dabei wurden 3.000 g AlOOH mit 150 g Kartoffelstärke und 28 g Methylcellulose und etwa 1 l Wasser angemaischt. Nach 3 h Knetzeit wurde die plastische Masse zu Wagenräder-Katalysatorträgern extrudiert und geschnitten. Die Katalysatorträger werden 1 Tag bei 150 °C entwässert und bei 1.100 °C 1 h lang calciniert. Durch dieses Verfahren wurden siliciumfreie Katalysatorträger einfacher Geometrie erhalten, die folgende Eigenschaften besitzen:

| | |
|---|---|
| - Seitendruckfestigkeit | 304 N |
| - Wasseraufnahme | 0,270 ml/g |
| - BET | 2,3 m²/g |
| - mittlerer Porendurchmesser (nach der Hg-Druckporosimetrie-Methode) | 0,56 µm |
| - Gesamtporenvolumen (nach der Hg-Druckporosimetrie-Methode) | 0,24 ml/g |

Nach Tränkung und Trocknung der im Extrudierverfahren hergestellten Wagenräder wie in Beispiel 1 wurden sie wie in Beispiel 1 bezüglich ihrer Performance bei der Synthese von N-Vinylformamid aus N-Formylalaninnitril getestet.

| | |
|---|---|
| - Umsatz | 89% |
| - Selektivität | 91% |
| - Druckverlust | 3,5 mbar |
| - Kaliumverlust | 26% (nicht als Silikat) |
| - Wärmedurchgangszahl | 14 W/m²K |
| - radiale Wärmeleitfähigkeit | 0,15 W/mK |
| - Standzeit | 600 h |

## Patentansprüche

1. SiO₂-freier, α-Al₂O₃ enthaltender Katalysatorträger in Form eines Hohlkörpers, dadurch gekennzeichnet, daß mindestens ein Teil der Außenwände des Hohlkörpers durchbrochen ist.

2. Katalysatorträger nach Anspruch 1, der sich entlang einer Längsachse erstreckt, dadurch gekennzeichnet, daß er sich entlang der Längsachse verjüngt.

3. Katalysatorträger nach Anspruch 2, dadurch gekennzeichnet, daß die Durchbrechungen der Außenwände des Hohlkörpers parallel zur Längsachse orientiert sind.

4. SiO₂-freier, α-Al₂O₃ enthaltender Katalysator, umfassend einen Katalysatorträger nach mindestens einem der Ansprüche 1 bis 3 und, darauf aufgebracht, mindestens eine Alkalimetallverbindung oder mindestens eine Erdalkalimetallverbindung oder ein Gemisch davon.

5. Verfahren zur Herstellung eines Katalysatorträgers gemäß mindestens einem der Ansprüche 1 bis 3, das die folgenden Stufen umfaßt:
I) Verformen eines Gemischs, enthaltend
(A) ein Pulver mit einem Gehalt an α-Al₂O₃ von mindestens 95 Gew.-% und
(B) ein polymeres Bindemittel
II) Entfernen des polymeren Bindemittels (B), und
III) Vorsintern des aus Stufe (II) erhaltenen Formkörpers.

6. Verfahren nach Anspruch 5, wobei das in Stufe (I) verformte Gemisch zusätzlich als Komponente
C) ein Dispergierhilfsmittel enthält, das in der Stufe (II) oder (III) entfernt wird.

7. Verfahren zur Herstellung eines Katalysators gemäß Anspruch 4, das zusätzlich zu den Stufen I) bis III), wie in Anspruch 5 oder 6 definiert, die Stufe
IV) Imprägnieren des aus Stufe (III) erhaltenen Formkörpers mit mindestens einer Lösung mindestens eines Alkalimetallsalzes oder mindestens eines Erdalkalimetallsalzes oder eines Gemisches davon und anschließendes Trocknen des erhaltenen Katalysators umfaßt.

8. SiO₂-freier, α-Al₂O₃ enthaltender Katalysator umfassend einen Katalysatorträger und mindestens eine Alkalimetallverbindung oder mindestens eine Erdalkalimetallverbindung oder ein Gemisch davon, herstellbar durch ein Verfahren, das die folgenden Stufen umfaßt:
I) Verformen eines Gemischs, enthaltend
(A) ein Pulver mit einem Gehalt an α-Al₂O₃ von mindestens 95 Gew.-% und
(B) ein polymeres Bindemittel
II) Entfernen des polymeren Bindemittels (B),
III) Vorsintern des aus Stufe (II) erhaltenen Formkörpers, und
IV) Imprägnieren des aus Stufe (III) erhaltenen Formkörpers mit mindestens einer Lösung mindestens eines Alkalimetallsalzes oder mindestens eines Erdalkalimetallsalzes oder eines Gemisches davon und anschließendes Trocknen des erhaltenen Katalysators.

9. Verwendung des Katalysators gemäß Anspruch 4 oder Anspruch 8 als Dehydrocyanisierungskatalysator.

10. Verwendung nach Anspruch 9, wobei bei der Dehydrocyanisierung N-Formylalaninnitril zu N-Vinylformamid umgesetzt wird.
